# EUROPEAN PATENT APPLICATION

(11) **EP 4 610 359 A1**
(43) Date of publication of application: **03.09.2025**
(21) Application number: 23886241.1
(22) Date of filing: 01.11.2023
(51) Int. Cl.: C12N 15/77, C07K 14/34, C12P 19/32, C12R 1/15

(54) **MICROORGANISM PRODUCING PURINE NUCLEOTIDES, AND METHOD FOR PRODUCING PURINE NUCLEOTIDES USING SAME**

(30) Priority: 01.11.2022 KR 20220143842
(71) Applicant: CJ CheilJedang Corporation, Seoul 04560 (KR)
(72) Inventor: KWON, Hee Su, Seoul 04560 (KR); BONG, Hyunju, Seoul 04560 (KR); BAE, Hyun-jung, Seoul 04560 (KR); LEE, Ji Hyun, Seoul 04560 (KR); LEE, Ji Hye, Seoul 04560 (KR)
(74) Representative: Meissner Bolte Partnerschaft mbB
(86) International application number: PCT/KR2023/017191
(87) International publication number: WO 2024/096545

(57) **Abstract**

The present disclosure relates to a microorganism producing purine nucleotides and a method of producing purine nucleotides using the same.

## Description

### [Technical Field]

The present disclosure relates to a microorganism producing purine nucleotides and a method of producing purine nucleotides using the same.

### [Background Art]

Purine nucleotides, which are intermediates in the nucleic acid biosynthesis metabolic systems, have a physiologically important role in the body and are widely used in foods, pharmaceuticals, *etc.* The purine nucleotides include 5'-inosine monophosphate (IMP), 5'-xanthosine monophosphate (XMP), 5'-guanosine monophosphate (GMP), *etc.*

For the production of purine nucleotides such as IMP, XMP, GMP, *etc.,* various studies have been conducted to develop microorganisms with high-efficiency production and fermentation process technology. For example, target substance-specific approaches are mainly used, such as increasing the expression of genes encoding enzymes involved in biosynthesis of IMP, XMP, GMP, *etc.,* or removing unnecessary genes in the biosynthesis (EP 3722430 A1, US 2020-0347346 A1).

However, when the purine nucleotides are produced from microorganisms producing the same through aerobic fermentation, there has been a problem that productivity decreases due to oxidative stress in the later stage of fermentation when the culture continues for a long time.

### [Disclosure]

### [Technical Problem]

The present inventors found that when an Mn transporter with enhanced activity is introduced into a microorganism, its purine nucleotide-producing ability is increased, as compared to the existing unmodified microorganism, thereby completing the present disclosure.

### [Technical Solution]

An object of the present disclosure is to provide a purine nucleotide-producing microorganism of the genus *Corynebacterium,* in which activity of an Mn transporter is enhanced.

Another object of the present disclosure is to provide a method of producing purine nucleotides, the method including the step of culturing, in a medium, a purine nucleotide-producing microorganism of the genus *Corynebacterium,* in which activity of an Mn transporter is enhanced.

Still another object of the present disclosure is to provide a composition for producing purine nucleotides, the composition including a microorganism of the genus *Corynebacterium,* in which activity of an Mn transporter is enhanced; a medium in which the microorganism is cultured; or a combination thereof.

### [Advantageous Effects]

A microorganism of the present disclosure may have increased purine nucleotide-producing ability by enhancing activity of an Mn transporter, as compared to existing unmodified microorganisms.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a purine nucleotide-producing microorganism of the genus *Corynebacterium,* in which activity of an Mn transporter is enhanced.

As used herein, the term "Mn transporter" is a protein that transports manganese (Mn), and belongs to an iron/manganese/zinc ABC (TroA-like ATP-binding cassette) transporter.

The amino acid sequence of the Mn transporter may be available in the NCBI GenBank, *etc.,* which is a known database.

For example, the Mn transporter of the present disclosure may be derived from a microorganism. The microorganism may be specifically derived from a microorganism of the genus *Corynebacterium,* and more specifically derived from *Corynebacterium stationis, Corynebacterium* sp. J010B-136, *Corynebacterium casei, Corynebacterium ammoniagenes, etc.,* but is not limited thereto.

For another example, the amino acid sequence of the Mn transporter of the present disclosure may be WP_066796334.1 derived from *Corynebacterium stationis* ATCC6872, but it is obvious that proteins having the Mn transporter activity, which are derived from various origins, are included.

In the present disclosure, the Mn transporter may have, may include, or may consist of an amino acid sequence of SEQ ID NO: 1, or may essentially consist of the amino acid sequence.

In the present disclosure, the amino acid sequence of SEQ ID NO: 1 may include an amino acid sequence having at least 70% or more, 75% or more, 76% or more, 85% or more, 90% or more, 95% or more, 96% or more, 97% or more, 98% or more, 99% or more, 99.5% or more, 99.7% or more, or 99.9% or more homology or identity to the amino acid sequence of SEQ ID NO: 1. Further, it is apparent that any protein having an amino acid sequence having deletion, modification, substitution, conservative substitution, or addition in part of the sequence may also be included within the scope of the present disclosure as long as it has the homology or identity and exhibits efficacy corresponding to that of the protein including the amino acid sequence of SEQ ID NO: 1.

For example, there may be sequence addition or deletion, naturally occurring mutation, silent mutation, or conservative substitution, which does not alter functions of the protein of the present disclosure at the N-terminus, C-terminus, and/or inside of the amino acid sequence.

The "conservative substitution" refers to a substitution of one amino acid with another amino acid having similar structural and/or chemical properties thereto. Such an amino acid substitution may generally occur on the basis of similarities in polarity, charge, solubility, hydrophobicity, hydrophilicity, and/or amphipathic nature of residues. Usually, the conservative substitution may have little or no effect on the activity of a protein or a polypeptide.

As used herein, the term "homology" or "identity" refers to the degree of similarity between two given amino acid sequences or nucleotide sequences and may be expressed as a percentage. The terms 'homology and identity' may be often used interchangeably.

The sequence homology or identity of conserved polynucleotides or polypeptides may be determined by a standard alignment algorithm, and default gap penalties established by a program to be used may be used together. Substantially, homologous or identical sequences may generally hybridize with each other as a whole or in part under moderate or highly stringent conditions. It is obvious that the hybridization also includes hybridization with a polynucleotide containing usual codons or codons considering codon degeneracy in the polynucleotide.

Whether any two polynucleotide or polypeptide sequences have homology, similarity, or identity may be determined by, for example, a known computer algorithm such as the "FASTA" program using default parameters as in Pearson et al (1988)[Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL/.](1988) SIAM J Applied Math 48: 1073). For example, homology, similarity, or identity may be determined using BLAST or ClustalW of the National Center for Biotechnology Information.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a binary comparison matrix (containing a value 1 for identity and a value 0 for non-identity) and the weighted comparison matrix of Gribskov et al(1986) Nucl. Acids Res. 14: 6745 as disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

The Mn transporter of the present disclosure may be encoded by znuA1 gene.

For example, znuA1 gene may be a polynucleotide encoding WP_066796334.1 derived from *Corynebacterium stationis* ATCC6872, but is not limited thereto. For another example, znuA1 gene may be a part of Ncam2275 or CP016326.1 derived from *Corynebacterium stationis* ATCC6872, but is not limited thereto, and it is obvious that znuA1 genes encoding the protein having the Mn transporter activity, which are derived from various origins, are included.

As used herein, the term "polynucleotide" refers to a DNA or RNA strand having more than a certain length as a nucleotide polymer which is a long chain of nucleotide monomers linked by covalent bonds, and more specifically, refers to a polynucleotide fragment encoding the protein.

The polynucleotide encoding the Mn transporter of the present disclosure may include a nucleotide sequence encoding the amino acid sequence of SEQ ID NO: 1. For example of the present disclosure, the polynucleotide of the present disclosure may have or may include a nucleotide sequence of SEQ ID NO: 2. Further, the polynucleotide of the present disclosure may consist of or may essentially consist of the nucleotide sequence of SEQ ID NO: 2. Specifically, the znuA1 gene may be encoded by a polynucleotide represented by the nucleotide sequence of SEQ ID NO: 2.

The polynucleotide of the present disclosure may be variously modified in a coding region thereof within a range in which the amino acid sequence of the Mn transporter is not changed, considering codon degeneracy or codons preferred by an organism in which the Mn transporter of the present disclosure is to be expressed. Specifically, the polynucleotide of the present disclosure may have or may include a nucleotide sequence having 70% or more, 75% or more, 76% or more, 85% or more, 90% or more, 95% or more, 96% or more, 97% or more, 98% or more, or 99% or more homology or identity to the sequence of SEQ ID NO: 2, or may consist or may essentially consist of a nucleotide sequence having 70% or more, 75% or more, 76% or more, 85% or more, 90% or more, 95% or more, 96% or more, 97% or more, 98% or more, or 99% or more homology or identity to the sequence of SEQ ID NO: 2, but is not limited thereto.

In addition, the polynucleotide of the present disclosure may include, without limitation, a sequence that is able to hybridize, under stringent conditions, with a probe capable of being prepared from a known gene sequence, for example, a sequence complementary to a part or the entirety of the polynucleotide sequence of the present disclosure. The term "stringent conditions" refers to a condition that enables specific hybridization between polynucleotides. These conditions are described in detail in a literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, such conditions may include conditions under which polynucleotides having high homology or identity, such as polynucleotides having 70% or more, 75% or more, 76% or more, 85% or more, 90% or more, 95% or more, 96% or more, 97% or more, 98% or more, or 99% or more homology or identity therebetween, hybridize with each other, but polynucleotides having lower homology or identity do not hybridize with each other, or washing conditions for common Southern hybridization, in which washing is conducted at a salt concentration and a temperature, corresponding to 60°C, 1×SSC, 0.1% SDS, specifically 60°C, 0.1 ×SSC, 0.1% SDS, and more specifically 68°C, 0.1 ×SSC, 0.1% SDS, once, specifically, twice or three times.

Hybridization requires that two nucleic acids have complementary sequences, although mismatches between bases may be possible depending on hybridization stringency. The term "complementary" is used to describe the relationship between nucleotide bases that are able to hybridize with each another. For example, as for DNA, adenine is complementary to thymine and cytosine is complementary to guanine. Therefore, the polynucleotide of the present disclosure may also include isolated nucleic acid fragments complementary to the entire sequence as well as substantially similar nucleic acid sequences.

Specifically, a polynucleotide having homology or identity to the polynucleotide of the present disclosure may be detected using hybridization conditions including a step of hybridization at Tm of 55°C and utilizing the above-described conditions. In addition, the Tm value may be 60°C, 63°C, or 65°C, but is not limited thereto, and may be appropriately controlled by a person skilled in the art according to the purpose.

The appropriate stringency for hybridizing polynucleotides depends on the length of the polynucleotides and the degree of complementarity, and variables thereof are well known in the art (*e.g*., Sambrook et al., supra).

As used herein, the term "microorganism (or strain)" encompasses all of wild-type microorganisms or naturally or artificially genetically modified microorganisms, refers to a microorganism in which a particular mechanism is enhanced or diminished due to the insertion of an exogenous gene or the enhancement or diminishment of activity of an endogenous gene, and may be a microorganism including a genetic modification for production of a target polypeptide, protein, or product.

Accordingly, the microorganism of the present disclosure may be exemplified by a recombinant microorganism having a genetic modification that enhances the activity of Mn transporter.

The microorganism of the present disclosure may have the enhanced activity of Mn transporter, as compared to the intrinsic activity.

For example, the microorganism of the present disclosure may have the enhanced activity of Mn transporter by increasing the expression of Mn transporter, as compared to the wild-type microorganism of the genus *Corynebacterium,* but is not limited thereto.

As used herein, the term "enhancement" of a polypeptide (Examples include proteins specified by the name of each enzyme) refers to an increase in activity of the polypeptide, as compared to the intrinsic activity thereof. The enhancement may be used interchangeably with activation, up-regulation, overexpression, increase, *etc.* Herein, the activation, enhancement, up-regulation, overexpression, and increase may include all of exhibiting the activity that has not been originally possessed or exhibiting the activity enhanced, as compared to the intrinsic activity or activity before modification. The "intrinsic activity" refers to the activity of a specific polypeptide originally possessed by a parental strain before transformation thereof or an unmodified microorganism, when transformed by genetic mutation caused by natural or artificial factors. This term may be used interchangeably with the "activity before modification". The "enhancement", "up-regulation", "overexpression", or "increase" in the activity of a polypeptide as compared to the intrinsic activity thereof means an improvement in activity and/or concentration (expression level) of a specific polypeptide originally possessed by a parent strain before transformation or an unmodified microorganism.

The enhancement may be achieved through the introduction of an exogenous polypeptide or the activity enhancement and/or concentration (expression level) of an endogenous polypeptide. The enhancement of the activity of the polypeptide may be identified by an increase in the activity degree or expression level of the corresponding polypeptide or the amount of a product produced from the corresponding polypeptide.

With respect to the objects of the present disclosure, the microorganism of the present disclosure may be a microorganism having the enhanced purine nucleotide-producing ability by enhancing the activity of Mn transporter. The unmodified microorganism in which the Mn transporter is not enhanced, which is the target strain to compare whether or not the purine nucleotide-producing ability or Mn transporter is increased, may be *Corynebacterium stationis* KCCM12151P (CJI0323, Korean Patent No. 10-1904675) which is a 5'-inosine monophosphate (IMP)-producing strain, *Corynebacterium stationis* KCCM12285P (CJX1664, Korean Patent No. 10-1950141) and *Corynebacterium stationis* KCCM12313P (CJX1682, Korean Patent No. 10-2006977) which are 5'-xanthosine monophosphate (XMP)-producing strains, but is not limited thereto.

The enhancement of the activity of the polypeptide may be achieved by applying various methods well known in the art, and is not limited as long as the method is able to enhance the activity of the target polypeptide, as compared to the microorganism before modification. Specifically, genetic engineering and/or protein engineering well known to a person skilled in the art, which is a routine method of molecular biology, may be used, but is not limited thereto (*e.g*., Sitnicka et al. Functional Analysis of Genes. Advances in Cell Biology. 2010, Vol. 2. 1-16, Sambrook et al. Molecular Cloning 2012, *etc*.)*.*

Specifically, the enhancement of the polypeptide of the present disclosure may be achieved by:
1) increase in intracellular copy number of the polynucleotide encoding the polypeptide;
2) replacement of a gene expression control region on the chromosome encoding the polypeptide with a sequence having strong activity;
3) modification of a nucleotide sequence encoding an initiation codon or 5'-UTR region of a gene transcript encoding the polypeptide;
4) modification of the amino acid sequence of the polypeptide to enhance activity of the polypeptide;
5) modification of the polynucleotide sequence encoding the polypeptide to enhance the activity of the polypeptide (e.g., modification of the polynucleotide sequence of the polypeptide gene to encode a polypeptide modified to enhance activity of the polypeptide);
6) introduction of an exogenous polypeptide exhibiting activity of the polypeptide or an exogenous polynucleotide encoding the same;
7) codon optimization of the polynucleotide encoding the polypeptide;
8) modification or chemical modification of an exposed site selected by analysis of a tertiary structure of the polypeptide; or
9) combination of two or more selected from 1) to 8), but is not particularly limited thereto.

More specifically, 1) the increase in intracellular copy number of the polynucleotide encoding the polypeptide may be achieved by the introduction, into a host cell, of a vector to which the polynucleotide encoding the corresponding polypeptide is operably linked and which is able to replicate and function independently of the host. Alternatively, 1) the increase may be achieved by the introduction of one or two or more copies of the polynucleotide encoding the corresponding polypeptide into the chromosome in a host cell. The introduction into the chromosome may be performed by the introduction, into a host cell, of a vector capable of inserting the polynucleotide into the chromosome in the host cell, but is not limited thereto. The vector is as described above.

2) The replacement of a gene expression control region (expression control sequence) on the chromosome encoding the polypeptide with a sequence with strong activity may be, for example, the mutation on the sequence through deletion, insertion, non-conservative or conservative substitution, or a combination thereof, or the replacement with a sequence having stronger activity, to further enhance the activity of the expression control region. The expression control region may include, but is not particularly limited to, a promoter, an operator sequence, a sequence encoding a ribosome binding site, a sequence controlling the termination of transcription and translation, *etc.* For example, it may be the replacement of the original promoter with a stronger promoter, but is not limited thereto.

Examples of the known stronger promoter may include CJ1 to CJ7 promoters (Korean Patent No. 10-0620092, US 7662943 B2), lac promoter, trp promoter, trc promoter, tac promoter, lamda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but are not limited thereto.

3) The modification of a nucleotide sequence encoding an initiation codon or 5'-UTR region of the gene transcript encoding the polypeptide may be, for example, the substitution with a nucleotide sequence encoding, rather than an endogenous initiation codon, another initiation codon having a higher expression rate of a polypeptide, but is not limited thereto.

4) and 5) The modification of the amino acid sequence or the polynucleotide sequence may be the mutation on the sequence through deletion, insertion, non-conservative or conservative substitution, or a combination thereof in the amino acid sequence of the polypeptide or the polynucleotide sequence encoding the polypeptide, or the replacement with an amino acid sequence or polynucleotide sequence modified to have stronger activity or an amino acid sequence or polynucleotide sequence modified to have increased activity, in order to enhance activity of the polypeptide, but is not limited thereto. Specifically, the replacement may be performed by inserting the polynucleotide into the chromosome by homologous recombination, but is not limited thereto. The vector used herein may further include a selection maker for checking the insertion of the chromosome.

6) The introduction of an exogenous polynucleotide exhibiting activity of the polypeptide may be the introduction, into a host cell, of an exogenous polynucleotide encoding a polypeptide exhibiting the same/similar activity with regard to the polypeptide. The exogenous polynucleotide is not limited to the origin or sequence thereof as long as the exogenous polynucleotide exhibits the same/similar activity with regard to the polypeptide. The method used in the introduction may be performed by any known transformation method appropriately selected by a person skilled in the art, and through the expression of the introduced polynucleotide in the host cell, the polypeptide is produced and the activity thereof may be enhanced.

7) The codon optimization of the polynucleotide encoding the polypeptide may be the codon optimization of an endogenous polynucleotide to increase transcription or translation thereof in a host cell, or the codon optimization of an exogenous polynucleotide to allow optimized transcription or translation thereof in a host cell.

8) The modification or chemical modification of an exposed site selected by analysis of a tertiary structure of the polypeptide may be, for example, the modification or chemical modification of an exposed site to be modified or chemically modified by comparing sequence information of a polypeptide to be analyzed with a database that stores sequence information of existing proteins, determining a template protein candidate according to similarity of the sequences, and identifying the structure based thereon.

Such enhancement of the activity of the polypeptide may mean that the activity, concentration, or expression level of the corresponding polypeptide is increased relative to the activity or concentration of the polypeptide expressed in a wild-type or a microbial strain before modification, or that the amount of a product produced from the corresponding polypeptide is increased, but is not limited thereto.

For example, the enhancement of the activity of the Mn transporter of the present disclosure may be enhancement due to the increase in the intracellular copy number of the polynucleotide encoding the Mn transporter. For another example, the enhancement of the activity of the Mn transporter of the present disclosure may be enhancement due to the replacement of the promoter of the gene encoding the Mn transporter or due to modification of the promoter. For still another example, the enhancement of the activity of the Mn transporter of the present disclosure may be enhancement due to introduction of a polynucleotide exhibiting the activity of Mn transporter, but is not limited thereto.

The modification of a part or the entirety of the polynucleotide in the microorganism of the present disclosure may be induced by: (a) homologous recombination using a vector for chromosomal insertion in a microorganism or genome editing using engineered nuclease (*e.g*., CRISPRCas9), or and/or (b) the treatment with light, such as ultraviolet light and radiation, and/or chemicals, but is not limited thereto. The method of modifying a part or the entirety of the gene may include a method by DNA recombinant technology. For example, a nucleotide sequence or vector containing a nucleotide sequence homologous to a target gene may be introduced into the microorganism to bring about homologous recombination, resulting in deletion in a part or the entirety of the gene. The nucleotide sequence or vector to be introduced may include a dominant selection marker, but is not limited thereto.

The vector of the present disclosure may include a DNA construct containing the nucleotide sequence of a polynucleotide, encoding a target polypeptide, which is operably linked to a suitable expression control region (or expression control sequence) so as to express the target polypeptide in an appropriate host. The expression control region may include a promoter capable of initiating transcription, any operator sequence for controlling such transcription, a sequence encoding a suitable mRNA ribosomal binding site, and a sequence controlling the termination of transcription and translation. The vector, after transformation into an appropriate host cell, may replicate or function independently of the genome of the host, or may be integrated into the genome itself.

The vector used in the present disclosure is not particularly limited, and any vector known in the art may be used. Examples of the vector that is usually used may include native or recombinant plasmids, cosmids, viruses, and bacteriophages. For example, pWE15, M13, MBL3, MBL4, IXII, ASHII, APII, t10, t11, Charon4A, and Charon21A may be used as phage vectors or cosmid vectors, and pDZ-based, pBR-based, pUC-based, pBluescriptll-based, pGEM-based, pTZ-based, pCL-based, and pET-based vectors may be used as plasmid vectors. Specifically, pDZ, pDC, pDCM2, pACYC177, pACYC184, pCL, pECCG117, pUC19, pBR322, pMW118, pCC1BAC vectors may be used.

For example, a polynucleotide encoding a target polypeptide may be inserted into the chromosome through a vector for chromosomal insertion in a cell. The insertion of the polynucleotide into the chromosome may be performed using any method known in the art, for example, homologous recombination, but is not limited thereto. The vector may further include a selection maker for investigating the insertion into the chromosome. The selection marker is for selecting cells transformed with the vector, i.e., identifying the insertion of a target nucleic acid molecule, and markers imparting a selectable phenotype, such as drug resistance, auxotrophy, cytotoxic drug resistance, or expression of surface polypeptide may be used. Under the circumstances of the treatment with a selective agent, only the cells expressing selection markers may survive or express other phenotypic traits, so that the transformed cells may be selected.

As used herein, the term "transformation" means that a vector containing a polynucleotide encoding a target polypeptide is introduced into a host cell or microorganism to allow the polypeptide encoded by the polynucleotide to be expressed in the host cell. The transformed polynucleotide may include any polypeptide as long as it may be expressed in a host cell, regardless of whether it is inserted and located into the chromosome of the host cell or located outside of the chromosome. In addition, examples of the polynucleotide include DNA and/or RNA encoding a target polypeptide. The polynucleotide may be introduced in any form as long as the polynucleotide may be introduced and expressed in a host cell. For example, the polynucleotide may be introduced in the form of an expression cassette, which is a gene construct containing all factors required for self-expression. The expression cassette may generally include a promoter operably linked to the polynucleotide, a transcription termination signal, a ribosome binding site, and a translation terminal signal. The expression cassette may be an expression vector enabling self-replication. In addition, the polynucleotide may be introduced in the form as it is into the host cell to be operably linked to the sequence required for expression in the host cell, but is not limited thereto.

Further, as used above, the term "operably linked" refers to a functional linkage between a promoter sequence for initiating and mediating the transcription of the polynucleotide encoding the target polypeptide of the present disclosure and the polynucleotide sequence.

For another example of the present disclosure, the microorganism of the present disclosure may be a microorganism having the purine nucleotide-producing ability.

The microorganism of the present disclosure may be a microorganism having the enhanced purine nucleotide-producing ability.

As used herein, the term "purine nucleotide" refers to a nucleotide containing a purine-based structure. The purine nucleotide may be XMP, IMP, GMP, or AMP, and specifically IMP, XMP, or GMP, but is not limited thereto.

The "inosine 5'-monophosphate or 5'-inosinic acid (IMP)" and "guanosine-5'-monophosphate or 5'-guanylic acid (GMP)" of the present disclosure, which are intermediates in the nucleic acid biosynthesis metabolic systems, have a physiologically important role in the body and are widely used in foods, pharmaceuticals, *etc.* Specifically, IMP itself is known to impart a beef flavor, and GMP, derived from XMP, is known to impart a mushroom flavor. Both of the materials are known to enhance the flavor of monosodium glutamate (MSG), and thus have attracted much attention as a flavor-enhancing nucleic acid-based seasoning.

In an embodiment, GMP may be produced by conversion from XMP, but is not limited thereto. For example, the production amount of GMP may be increased by the conversion of GMP from the increasingly produced XMP, and thus the GMP is also included within the scope of the present disclosure.

The "XMP" of the present disclosure is also called xanthosine-5'-monophosphate or 5'-xanthylic acid, and may be formed from IMP through the action of IMP dehydrogenase or converted to GMP through the action of GMP synthase.

The microorganism of the present disclosure may be a microorganism in which the activity of Mn transporter is enhanced. Specifically, the microorganism of the present disclosure may be a microorganism in which the Mn transporter or znuA1 gene encoding the same is enhanced; or a microorganism (e.g., recombinant microorganism) which is genetically modified to enhance the Mn transporter or znuA1 gene encoding the same.

The microorganism of the present disclosure may be a microorganism naturally having the purine nucleotide-producing ability, or a microorganism in which the purine nucleotide-producing ability is increased or imparted by enhancing the Mn transporter or the polynucleotide encoding the same in a parent strain without the purine nucleotide-producing ability, but is not limited thereto.

With respect to the objects of the present disclosure, the recombinant microorganism of the present disclosure may be a microorganism having the increased purine nucleotide-producing ability by enhancing the Mn transporter or the polynucleotide encoding the same in a natural wild-type microorganism or in a microorganism producing purine nucleotides by including the Mn transporter or the polynucleotide encoding the same, as compared to the natural wild-type microorganism or the microorganism producing purine nucleotides by including the Mn transporter or the polynucleotide encoding the same, but is not limited thereto. For example, the natural wild-type microorganism or the microorganism producing purine nucleotides by including the Mn transporter or the polynucleotide encoding the same may be a target strain for comparing whether or not the purine nucleotide-producing ability or the Mn transporter is increased, as described above, but is not limited thereto.

For example, the recombinant strain having the increased producing ability may have an increased purine nucleotide-producing ability of about 1% or more, specifically, about 2% or more, about 5% or more, about 5.4% or more, about 5.7% or more, about 6% or more, about 6.7% or more, about 7% or more, about 8% or more, about 9% or more, about 10% or more, about 11% or more, about 11.5% or more, about 12% or more, about 12.9% or more, about 13% or more, about 14% or more, about 14.4% or more, about 15% or more, about 16% or more, about 17% or more, about 18% or more, about 19% or more, or about 20% or more (the upper limit is not particularly limited, but may be, for example, about 200% or less, about 150% or less, about 100% or less, about 50% or less, about 40% or less, about 30% or less, about 20% or less, or about 15% or less), as compared to that of the parent strain before modification or unmodified microorganism, but the increased amount is not limited thereto as long as the producing ability has an increased amount of a + value, as compared to the producing ability of the parent strain before modification or the unmodified microorganism. In another example, the microorganism having the increased producing ability may have an increased purine nucleotide-producing ability of about 1.01 times or more, about 1.02 times or more, about 1.05 times or more, about 1.054 times or more, about 1.057 times or more, about 1.06 times or more, about 1.067 times or more, about 1.07 times or more, about 1.08 times or more, about 1.09 times or more, about 1.10 times or more, about 1.11 times or more, about 1.115 times or more, about 1.12 times or more, about 1.129 times or more, about 1.13 times or more, about 1.14 times or more, about 1.144 times or more, about 1.15 times or more, about 1.16 times or more, about 1.17 times or more, about 1.18 times or more, about 1.19 times or more, or about 1.20 times or more (the upper limit is not particularly limited, but may be, for example, about 10 times or less, about 5 times or less, about 3 times or less, about 2 times or less), as compared to that of the parent strain before modification or the unmodified microorganism, but is not limited thereto.

Such producing ability may be evaluated by measuring the production amount of a target product which is obtained by culturing in a medium. The evaluation may be performed by measuring the production amount of a target product using an appropriate method known in the art. For example, high performance liquid chromatography (HPLC), gas chromatography (GC), gas chromatography-mass spectrometry (GC/MS), liquid chromatography-mass spectrometry (LC/MS), gel permeation chromatography (GPC), or a combination thereof may be used, and the production amount of a target product may be measured using an appropriate method known in the art.

As used herein, the term "unmodified microorganism" may refer to a wild-type strain or a native strain as it is, or a strain before transformation due to genetic mutation caused by natural or artificial factors, not excluding strains including mutations that may naturally occur in microorganisms. For example, the unmodified microorganism may refer to a strain to which the Mn transporter or the polynucleotide encoding the same described herein is not enhanced, or a strain before the enhancement thereof. The "unmodified microorganism" may be used interchangeably with "strain before modification", "microorganism before modification", "unvaried strain", "unmodified strain", "unvaried microorganism", or "reference microorganism".

In the present disclosure, the purine nucleotide may be any one or more selected from the group consisting of IMP, XMP, and GMP. Description thereof are as described above.

For still another example of the present disclosure, the microorganism of the present disclosure may be *Corynebacterium stationis, Corynebacterium glutamicum, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* specifically, *Corynebacterium stationis,* but is not limited thereto.

For still another example of the present disclosure, the recombinant microorganism of the present disclosure may be a microorganism in which the purine nucleotide-producing ability is enhanced by additionally enhancing the activity of some of the proteins involved in the purine nucleotide biosynthesis pathway, or by additionally diminishing the activity of some of the proteins involved in the purine nucleotide degradation pathway.

As used herein, the term "diminishment" of the activity of a polypeptide has a concept encompassing all of the reduction of activity or the absence of activity, as compared to the intrinsic activity. The diminishment may be used interchangeably with inactivation, deficiency, down-regulation, decrease, reduction, attenuation, or the like.

The diminishment may also include a case where the activity of the polypeptide itself is reduced or eliminated compared to the activity of the polypeptide possessed by the original microorganism due to the mutation, *etc.* of the polynucleotide encoding the polypeptide; a case where the activity and/or concentration (expression level) of the overall polypeptides in the cell is lower compared to the native strain due to the inhibition of the expression of a gene of a polynucleotide encoding the polypeptide or the inhibition of translation into the polypeptide; a case where the expression of the polynucleotide is not made; and/or a case where the polypeptide has no activity in spite of the expression of the polynucleotide. The "intrinsic activity" refers to the activity of a specific polypeptide originally possessed by a parent strain before modification or a wild-type or unmodified microorganism, when transformed by genetic mutation caused by natural or artificial factors. This term may be used interchangeably with the "activity before modification". The "diminishment, inactivation, deficiency, decrease, down-regulation, reduction, or attenuation" of the activity of a polypeptide compared to the intrinsic activity thereof means that the activity of the polypeptide is lowered compared to the activity of a specific polypeptide originally possessed by a parent strain before transformation or an unmodified microorganism.

The diminishment of the activity of the polypeptide may be performed by any method known in the art, but is not limited thereto, and may be achieved by applying various methods well known in the art (*e.g*., Nakashima N et al., Bacterial cellular engineering by genome editing and gene silencing. Int J Mol Sci. 2014;15(2):2773-2793, Sambrook et al. Molecular Cloning 2012, *etc.*)*.*

Specifically, the diminishment of the activity of the polypeptide of the present disclosure may be achieved by:
1) deletion of a part or the entirety of the gene encoding the polypeptide;
2) modification of an expression control region (or expression control sequence) to reduce the expression of a gene encoding the polypeptide;
3) modification of the amino acid sequence constituting the polypeptide to eliminate or diminish the activity of the polypeptide (*e.g.*, deletion/substitution/addition of one or more amino acids in the amino acid sequence);
4) modification of the gene sequence encoding the polypeptide to eliminate or diminish the activity of the polypeptide (*e*.*g*., deletion/substitution/addition of one or more nucleotides in the nucleotide sequence of the polypeptide gene to encode the polypeptide which is modified to eliminate or diminish the activity of the polypeptide);
5) modification of a nucleotide sequence encoding an initiation codon, a Shine-Dalgarno sequence, or 5'-UTR region of the gene transcript encoding the polypeptide;
6) introduction of an antisense oligonucleotide (*e.g*., antisense RNA) complementarily binding to the gene transcript encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE); or
9) combination of two or more selected from 1) to 8), but is not particularly limited thereto.

For example,
1) the deletion of a part or the entirety of the gene encoding the polypeptide may be the elimination of the entirety of the polynucleotide encoding an endogenous target protein in the chromosome, the replacement with a polynucleotide having deletion of some nucleotides, or the replacement with a marker gene.
2) The modification of an expression control region (or expression control sequence) may be the mutation on the expression control region (or expression control sequence) through deletion, insertion, non-conservative or conservative substitution, or a combination thereof, or the replacement with a sequence having more diminished activity. The expression control region includes a promoter, an operator sequence, a sequence for encoding a ribosomal binding site, and a sequence for controlling the termination of transcription and translation, but is not limited thereto.
3) and 4) The modification of the amino acid sequence or the polynucleotide sequence may be the mutation on the sequence through deletion, insertion, non-conservative or conservative substitution, or a combination thereof in the amino acid sequence of the polypeptide or the polynucleotide sequence encoding the polypeptide, or a replacement with an amino acid sequence or polynucleotide sequence modified to have more diminished activity or an amino acid sequence or polynucleotide sequence improved to have no activity, so as to diminish activity of the polypeptide, but is not limited thereto. For example, the expression of the gene may be inhibited or attenuated by introducing mutation into the polynucleotide sequence to form a termination codon, but is not limited thereto.
5) The modification of a nucleotide sequence encoding an initiation codon or 5'-UTR region of the gene transcript encoding the polypeptide may be, for example, the substitution with a nucleotide sequence encoding, rather than an endogenous initiation codon, another initiation codon having a lower expression rate of the polypeptide, but is not limited thereto.
6) The introduction of an antisense oligonucleotide (*e.g*., antisense RNA) complementarily binding to the gene transcript encoding the polypeptide may be referred to, for example, a literature [Weintraub, H. et al., Antisense-RNA as a molecular tool for genetic analysis, Reviews - Trends in Genetics, Vol. 1(1) 1986].
7) The addition of a sequence complementary to the Shine-Dalgarno sequence of the gene encoding the polypeptide to the upstream of the Shine-Dalgarno sequence so as to form a secondary structure that makes the attachment of ribosomes impossible may be making mRNA translation impossible or reducing the rate thereof.
8) The addition of a promoter, which is to be reverse-transcribed, to the 3' end of the open reading frame (ORF) of the gene sequence encoding the polypeptide (reverse transcription engineering, RTE) may be making an antisense nucleotide complementary to the gene transcript encoding the polypeptide to diminish the activity of the polypeptide.

Another aspect of the present disclosure provides a method of producing purine nucleotides, the method including the step of culturing, in a medium, a purine nucleotide-producing microorganism of the genus *Corynebacterium,* in which activity of an Mn transporter is enhanced.

The method of producing purine nucleotides of the present disclosure may include the step of culturing, in a medium, a microorganism in which the Mn transporter or znuA1 gene encoding the same is enhanced; or a microorganism which is genetically modified to have the enhanced Mn transporter or znuA1 gene encoding the same, and the microorganism is as described above.

As used herein the term "culture" refers to growing the microorganism of the present disclosure in appropriately adjusted environment conditions. The culture procedure of the present disclosure may be performed according to appropriate media or culture conditions known in the art. Such culture may be easily adjusted according to the selected microorganism by a person skilled in the art. Specifically, the culture may be in a batch type, a continuous type, and/or a fed-batch type, but is not limited thereto.

As used herein, the "medium" refers to a mixture containing, as main ingredients, nutrient materials required for culturing the microorganism of the present disclosure, wherein the medium supplies nutrient materials including water essential for survival and growth, growth factors, *etc.* Specifically, as for the media and other culture conditions used for culturing the microorganism of the present disclosure, any medium that is used for usual culture of microorganisms may be used without particular limitation. However, the microorganism of the present disclosure may be cultured under aerobic conditions in a common medium containing appropriate carbon sources, nitrogen sources, phosphorus sources, inorganic compounds, amino acids, and/or vitamins, while controlling the temperature, pH, *etc.*

Specifically, a culture medium for microorganisms of the genus *Corynebacterium* may be found in a literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* and amino acids, such as glutamic acid, methionine, lysine, *etc.* In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, *etc.* may be used. These nitrogen sources may be used alone or in a combination of two or more thereof, but are not limited thereto.

The phosphate sources may include potassium phosphate monobasic, potassium phosphate dibasic, and sodium-containing salts corresponding thereto. As for inorganic compounds, sodium chloride, calcium chloride, iron chloride, magnesium sulfate, iron sulfate, manganese sulfate, calcium carbonate, *etc.* may be used, and in addition, amino acids, vitamins, and/or suitable precursors may be included. These constituent ingredients or precursors may be added to the medium in a batch or continuous manner. However, the present disclosure is not limited thereto.

The pH of the medium may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be added to suppress foam formation during the culture. In addition, oxygen or oxygen-containing gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culture of the present disclosure, the culture temperature may be maintained at 20°C to 45°C, specifically 25°C to 40°C, and the culture may be performed for about 10 hours to 160 hours, but is not limited thereto.

The purine nucleotide produced by the culture of the present disclosure may be released into the medium or may remain in cells.

The purine nucleotide may be any one or more selected from the group consisting of IMP, XMP, and GMP.

The method of producing purine nucleotides of the present disclosure may further include the steps of preparing the microorganism of the present disclosure, preparing a medium for culturing the microorganism, or a combination of these steps (regardless of the order, in any order), for example, before or after the culturing step.

The method of producing purine nucleotides of the present disclosure may further include the step of recovering the purine nucleotide from a medium resulting from the culture (a medium in which culture has been performed) or the cultured microorganism. The recovering step may be further included after the culturing step.

The recovering may be collecting a desired purine nucleotide by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, and a desired purine nucleotide may be recovered from the medium or microorganism by using an appropriate method known in the art.

In addition, the method of producing purine nucleotides of the present disclosure may further include a purification step. The purification may be performed by using an appropriate method known in the art. In an exemplary embodiment, when the method of producing purine nucleotides of the present disclosure includes both the recovering step and the purification step, the recovering step and the purification step may be performed continuously or discontinuously regardless of the order, or may be performed simultaneously or integrated into one step, but is not limited thereto.

In the method of the present disclosure, the Mn transporter, polynucleotide, vector, microorganism, *etc.* are as described in other aspects.

Still another aspect of the present disclosure provides a composition for producing purine nucleotides, the composition including the microorganism of the genus *Corynebacterium,* in which the activity of Mn transporter is enhanced; a medium in which the microorganism is cultured; or a combination thereof.

The composition of the present disclosure may further include any appropriate excipient that is usually used in the composition for producing purine nucleotides, and examples of the excipient may include a preserving agent, a wetting agent, a dispersing agent, a suspending agent, a buffer, a stabilizing agent, an isotonic agent, *etc.,* but are not limited thereto.

Still another aspect of the present disclosure provides a method of preparing the purine nucleotide-producing microorganism of the genus *Corynebacterium,* the method including the step of enhancing the activity of Mn transporter.

The preparation method may include the step of modifying the microorganism to have the enhanced activity of Mn transporter, as compared to the intrinsic activity.

Still another aspect of the present disclosure provides use of the microorganism of the genus *Corynebacterium,* in which the activity of Mn transporter is enhanced, in the production of purine nucleotides.

The Mn transporter, enhancement, microorganism of the genus *Corynebacterium, etc.* are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Preparation of vector

### Example 1-1: Preparation of vector for increasing copy number of znuA1 gene

A vector for increasing the copy number of znuA1(Ncam2275) gene (CP016326.1, SEQ ID NO: 2) encoding an Mn transporter (Iron/manganese/zinc ABC (TroA-like ATP-binding cassette) transporter) (WP_066796334.1, SEQ ID NO: 1) was prepared.

In detail, chromosomal gene of the wild-type *Corynebacterium stationis* ATCC6872 strain was isolated using Intron's G-spin Total DNA extraction mini kit (Cat. No 17045) according to the protocol, and used as a template to perform PCR using a primer pair of SEQ ID NO: 3 and SEQ ID NO: 4, a primer pair of SEQ ID NO: 5 and SEQ ID NO:6, and a primer pair of SEQ ID NO: 7 and SEQ ID NO: 8, respectively. PCR conditions included denaturation at 94°C for 5 minutes, 20 cycles of denaturation at 94°C for 30 seconds, annealing at 55°C for 30 seconds, and polymerization at 72°C for 1 minute, and then polymerization at 72°C for 7 minutes. As a result, gene fragments of Pn/Ncam2275-A (1050bp), Pn/Ncam2275-B (1648bp), and Pn/Ncam2275-C (1048bp) were obtained, respectively.

Secondary PCR was performed using the three types of gene fragments thus obtained as a template under the same conditions to obtain 3.6k bp of a gene fragment. The obtained gene fragment was digested with a restriction enzyme Xbal (New England Biolabs, Beverly, MA), and cloned into a linear pDCM2 vector (Korean Patent No. 10-2020-0136813) which had been digested with the same restriction enzyme Xbal using T4 ligase (New England Biolabs, Beverly, MA). The prepared vector was named pDCM2-Pn/Ncam2275.

The primer sequences used in Example 1-1 are as in Table 1 below.

**[Table 1]**

| SEQ ID NO. | Name of sequence | Sequence (5'-> 3') |
|---|---|---|
| 3 | Pn/Ncam2275-A-F | AATTCGAGCTCGGTACCCTGGCAAACTCAAATCCGG CT |
| 4 | Pn/Ncam2275-A-R | TTTCTTAGCAGGTATGCGTAGGTCAAAGTATCGCC |
| 5 | Pn/Ncam2275-B-F | TACTTTGACCTACGCATACCTGCTAAGAAATCCAC |
| 6 | Pn/Ncam2275-B-R | GATTCGGTCTGCTTGAAGGATGGTTGGGCTTGCAT |
| 7 | Pn/Ncam2275-C-F | AGCCCAACCATCCTTCAAGCAGACCGAATCCATCG |
| 8 | Pn/Ncam2275-C-R | CGACTCTAGAGGATCCCCGTACCAAGCGTGAAGAGG TT |

### Example 1-2: Preparation of vector for increasing copy number of znuA1 gene and for substituting promoter

A vector for increasing the copy number of znuA1 gene and for substituting a native promoter with a pCJ7 promoter (Korean Patent No. 10-0620092) was prepared.

In detail, PCR was performed using the chromosomal gene of the wild-type *Corynebacterium stationis* ATCC6872 strain as a template and a primer pair of SEQ ID NO: 9 and SEQ ID NO: 10, a primer pair of SEQ ID NO: 11 and SEQ ID NO: 12, a primer pair of SEQ ID NO: 13 and SEQ ID NO: 14, and a primer pair of SEQ ID NO: 15 and SEQ ID NO: 16, respectively. PCR conditions included denaturation at 94°C for 5 minutes, 20 cycles of denaturation at 94°C for 30 seconds, annealing at 55°C for 30 seconds, and polymerization at 72°C for 1 minute, and then polymerization at 72°C for 7 minutes. As a result, gene fragments of Pcj7/Ncam2275-A (1050bp), Pcj7/Ncam2275-B (318bp), Pcj7/Ncam2275-C (1079bp), and Pcj7/Ncam2275-D (1048bp) were obtained, respectively.

Secondary PCR was performed using the four types of gene fragments thus obtained as a template under the same conditions to obtain 3.5k bp of a gene fragment. The obtained gene fragment was digested with a restriction enzyme Xbal and cloned into a linear pDCM2 vector which had been digested with the same restriction enzyme Xbal using T4 ligase. The prepared vector was named pDCM2-Pcj7/Ncam2275.

The primer sequences used in Example 1-2 are as in Table 2 below.

**[Table 2]**

| SEQ ID NO. | Name of sequence | Sequence (5'-> 3') |
|---|---|---|
| 9 | Pcj7/Ncam2275-A-F | AATTCGAGCTCGGTACCCTGGCAAACTCAAATCCGGC T |
| 10 | Pcj7/Ncam2275-A-R | cgctgggatgtttctGCGTAGGTCAAAGTATCGCC |
| 11 | Pcj7/Ncam2275-B-F | TACTTTGACCTACGCagaaacatcccagcgctact |
| 12 | Pcj7/Ncam2275-B-R | AGTCTGTAAATGCATgagtgtttcctttcgttggg |
| 13 | Pcj7/Ncam2275-C-F | cgaaaggaaacactcATGCATTTACAGACTTCTCA |
| 14 | Pcj7/Ncam2275-C-R | GATTCGGTCTGCTTGAAGGATGGTTGGGCTTGCAT |
| 15 | Pcj7/Ncam2275-D-F | AGCCCAACCATCCTTCAAGCAGACCGAATCCATCG |
| 16 | Pcj7/Ncam2275-D-R | CGACTCTAGAGGATCCCCGTACCAAGCGTGAAGAGGT T |

### Example 1-3: Preparation of vector for introducing Mn transporter

A vector for introducing Mn transporter was prepared.

In detail, PCR was performed using the chromosomal gene of the wild-type *Corynebacterium stationis* ATCC6872 strain as a template and a primer pair of SEQ ID NO: 17 and SEQ ID NO: 18, a primer pair of SEQ ID NO: 19 and SEQ ID NO: 20, and a primer pair of SEQ ID NO: 21 and SEQ ID NO: 22, respectively. PCR conditions included denaturation at 94°C for 5 minutes, 20 cycles of denaturation at 94°C for 30 seconds, annealing at 55°C for 30 seconds, and polymerization at 72°C for 1 minute, and then polymerization at 72°C for 7 minutes.

The obtained gene fragment was fusion-cloned into a linear pDCM2 vector which had been digested with a restriction enzyme Xbal using an In-Fusion^{®} HD cloning kit (Clontech). The prepared vector was named pDCM2-deINCam2155-NCam2275.

The primer sequences used in Example 1-3 are as in Table 3 below.

**[Table 3]**

| SEQ ID NO. | Name of sequence | Sequence (5'-> 3') |
|---|---|---|
| 17 | delNCam2155-NCam2275-A-F | ACGACGGCCAGTGAATTCGAGCTCGGTACCCGGGGATCCTgc gcggcaagaaggggacc |
| 18 | delNCam2155-NCam2275-A-R | TCACTGAAAGCTTTCACGTCCATCGCACTAGTGCGGCCGCag tttgcaccggctttgcca |
| 19 | delNCam2155-NCam2275-B-F | ACTAGTGCGATGGACGTGAAAGCT |
| 20 | delNCam2155-NCam2275-B-R | GACCATGATTACGCCAAGCTTGCATGCCTGCAGGTCGACTgct aatgcgccctgggaatg |
| 21 | delNCam2155-NCam2275-C-F | AAAGCCGGTGCAAACTGCCATACCTGCTAAGAAATCCACC |
| 22 | delNCam2155-NCam2275-C-R | CGTCCATCGCACTAGTGCAAGGATGGTTGGGCTTGCATC |

### Example 2: Preparation of purine nucleotide-producing strain

### Example 2-1: Preparation of strain with increased copy number of znuA1 gene

To prepare a purine nucleotide-producing strain, the pDCM2-Pn/Ncam2275 vector prepared in Example 1-1 was transformed into *Corynebacterium stationis* KCCM12151P (CJI0323, Korean Patent No. 10-1904675) which is a 5'-inosine monophosphate (IMP)-producing strain by electroporation (Appl. Microbiol. Biotechnol. (1999) 52:541-545), and then the strain having the vector inserted into the chromosome was subjected to primary selection on a selection medium containing 25 mg/L kanamycin. Then, a strain, in which the copy number of endogenous znuA1 gene on the chromosome was increased to 2 copies, was obtained through a secondary cross-over process using a homology between the existing gene on the chromosome and the gene inserted through the vector. The strain was subjected to PCR using a primer pair of SEQ ID NO: 23 and SEQ ID NO: 24, and finally selected through gene sequence analysis. The strain thus obtained, in which the copy number of znuA1 gene was increased to 2 copies, was named KCCM12151P_Pn/Ncam2275.

### Example 2-2: Preparation of strain with increased copy number of znuA1 gene and substitution of promoter

To prepare a purine nucleotide-producing strain, in which znuA1 gene is enhanced, as compared to the strain of Example 2-1, the pDCM2-Pcj7/Ncam2275 vector prepared in Example 1-2 was transformed into *Corynebacterium stationis* KCCM12151P which is an IMP-producing strain by electroporation, and then the strain having the vector inserted into the chromosome was subjected to primary selection on a selection medium containing 25 mg/L kanamycin. Then, a strain, in which the copy number of endogenous znuA1 gene on the chromosome was increased to 2 copies and the promoter was replaced by pCJ7 promoter, was obtained through a secondary cross-over process using a homology between the existing gene on the chromosome and the gene inserted through the vector. The strain was subjected to PCR using a primer pair of SEQ ID NO: 23 and SEQ ID NO: 24, and finally selected through gene sequence analysis. The strain thus obtained, in which the copy number of znuA1 gene was increased to 2 copies and the promoter was replaced by pCJ7 promoter, was named KCCM12151P_Pcj7/Ncam2275.

The primer sequences used in Examples 2-1 and 2-2 are as in Table 4 below.

**[Table 4]**

| SEQ ID NO. | Name of sequence | Sequence (5'-> 3') |
|---|---|---|
| 23 | Pn/Ncsm2275-confirm_F | GTCTCGTCCATCGATGGCAA |
| 24 | Pn/Ncsm2275-confirm_R | GCTGGCGACAACTGTGGTCT |

### Example 2-3: Preparation of Mn transporter-introduced strain

To prepare a purine nucleotide-producing strain, the pDCM2-delNCam2155-NCam2275 vector prepared in Example 1-3 was transformed into *Corynebacterium stationis* KCCM12285P (CJX1664, Korean Patent No. 10-1950141) and *Corynebacterium stationis* KCCM12313P (CJX1682, Korean Patent No. 10-2006977) which are 5'-xanthosine monophosphate (XMP)-producing strains by electroporation, respectively, and each strain having the vector inserted into the chromosome was subjected to primary selection on a selection medium containing 25 mg/L kanamycin. Then, a strain, in which the Mn transporter was introduced into the chromosome, was obtained through a secondary cross-over process using a homology between the existing gene on the chromosome and the gene inserted through the vector. The strain was subjected to PCR using a primer pair of SEQ ID NO: 17 and SEQ ID NO: 22, and finally selected through gene sequence analysis. The strains thus obtained, in which the Mn transporter was introduced, were named CJX2591 (CJX1664_deINCam2155-NCam2275) and CJX2592 (CJX1682_delINCam2155-NCam2275), respectively.

### Example 3: Evaluation of IMP-producing ability of strain

To measure the IMP-producing ability of KCCM12151P_Pn/Ncam2275 strain prepared in Example 2-1, KCCM12151P_Pcj7/Ncam2275 strain prepared in Example 2-2, and a control strain *Corynebacterium stationis* KCCM12151P, a flask fermentation titer test was performed.

Each strain was inoculated in a 14 mL tube containing 2.5 mL of the following seed medium, and cultured at 30°C with shaking at 170 rpm for 24 hours. In a 250 mL corner-baffle flask containing 24 mL of the following fermentation medium (24 mL of a main medium + 5 mL of a separate sterile medium), 2 mL of the seed culture was inoculated and cultured at 30°C with shaking at 170 rpm for 75 hours. After completion of the culture, IMP concentrations were measured using HPLC.

The compositions of the seed medium and fermentation medium are as follows.

### <IMP seed medium>

1% glucose, 1% peptone, 1% meat extract, 1% yeast extract, 0.25% sodium chloride, 100 mg/L adenine, 100 mg/L guanine, pH 7.2

### <IMP flask fermentation medium (main medium)>

0.1% sodium glutamate, 1% ammonium chloride, 1.2% magnesium sulfate, 0.01% calcium chloride, 20 mg/L iron sulfate, 20 mg/L manganese sulfate, 20 mg/L zinc sulfate, 5 mg/L copper sulfate, 23 mg/L L-cysteine, 24 mg/L beta-alanine, 8 mg/L nicotinic acid, 45 µg/L biotin, 5 mg/L thiamine hydrochloride, 30 mg/L adenine, 1.9% phosphoric acid (85%), 4.2% glucose, 2.4% fructose

### <IMP flask separate sterile medium>

23.3 g/L phosphoric acid (85%), 16.25 g/L ammonia (28%), 25.5 g/L potassium hydroxide

The above experiments were performed in triplicate, and the average values of the analysis results are shown in Table 5 below.

**[Table 5]**

| Name of strain | Concentration of residual sugar(g/L) | OD | IMP concentration (g/L) |
|---|---|---|---|
| KCCM12151P | 18.3 | 41.3 | 5.2 |
| KCCM12151P_Pn/Ncam2275 | 16.1 | 43.6 | 5.5 |
| KCCM12151P_Pcj7/Ncam2275 | 13.9 | 45.5 | 5.8 |

As shown in Table 5, it was confirmed that KCCM12151P_Pn/Ncam2275 strain and KCCM12151P_Pcj7/Ncam2275 strain, in which znuA1 gene was enhanced, showed 12% and 24% reduction in the amount of residual sugar in proportion to the degree of znuA1 gene enhancement, thereby exhibiting improvement in the sugar consumption rate, and showed 5.7% and 11.5% increase in the IMP production amount, respectively, as compared to the control strain.

The KCCM12151P_Pn/Ncam2275 strain and the KCCM12151P_Pcj7/Ncam2275 strain were named CJI-3141 and CJI-3142, respectively, which were deposited at the Korea Microorganism Conservation Center, a depository institution under the Budapest Treaty, on September 8, 2022, and given accession number KCCM13234P and KCCM13235P, respectively.

### Example 4: Evaluation of XMP-producing ability of strain

To measure the XMP-producing ability of CJX2591 (CJX1664_deINCam2155-NCam2275) strain and CJX2592 (CJX1682_deINCam2155-NCam2275) strain prepared in Example 2-3, and control strains *Corynebacterium stationis* KCCM12285P (CJX1664) and *Corynebacterium stationis* KCCM12313P (CJX1682), a flask fermentation titer test was performed.

Each strain was inoculated in a test tube with a diameter of 18 mm containing 2 mL of the following seed medium, and cultured at 30°C with shaking at 170 rpm for 24 hours. In a 250 mL corner-baffle flask containing 32 mL of the following fermentation medium (24 mL of a main medium + 8 mL of a separate sterile medium), 0.7 mL of the seed culture was inoculated and cultured at 30°C with shaking at 170 rpm for 75 hours. After completion of the culture, XMP concentrations were measured using HPLC.

The compositions of the seed medium and fermentation medium are as follows.

### <XMP seed medium>

1% glucose, 1% peptone, 1% meat extract, 1% yeast extract, 0.25% sodium chloride, 100 mg/L adenine, 100 mg/L guanine, pH 7.5

### <XMP flask fermentation medium (main medium)>

40 g/L glucose, 10 g/L magnesium sulfate, 100 mg/L calcium chloride, 20 mg/L iron sulfate, 10 mg/L manganese sulfate, 10 mg/L zinc sulfate, 0.8 mg/L copper sulfate, 20 mg/L histidine, 15 mg/l cystine, 15 mg/L beta-alanine, 100 µg/L biotin, 5 mg/L thiamine, 50 mg/L adenine, 25 mg/L guanine, 15 mg/L niacin, pH 7.0

### <XMP flask separate sterile medium>

18 g/L potassium phosphate monobasic, 42 g/L potassium phosphate dibasic, 7 g/L urea, 5 g/L ammonium sulfate

The above experiments were performed in triplicate, and the average values of the analysis results are shown in Table 6 below.

**[Table 6]**

| Name of strain | XMP concentration (g/L) | Increase rate of XMP concentration (%) |
|---|---|---|
| CJX1664 | 4.62 | - |
| CJX2591 | 5.22 | 12.98 % |
| CJX1682 | 12.56 | - |
| CJX2592 | 13.24 | 5.41 % |

As shown in Table 6, it was confirmed that CJX2591 strain and CJX2592 strain showed 12.9% and 5.4% increase in the XMP-producing ability, respectively, as compared to the control CJX1664 and CJX1682 strains.

### Example 5: Evaluation of GMP-producing ability of strain

To measure the 5'-guanosine monophosphate (GMP)-producing ability of CJX2591 (CJX1664_deINCam2155-NCam2275) strain and CJX2592 (CJX1682_deINCam2155-NCam2275) strain prepared in Example 2-3, and control strains *Corynebacterium stationis* KCCM12285P (CJX1664) and *Corynebacterium stationis* KCCM12313P(CJX1682), a flask fermentation titer test was performed in the same manner as in Example 4. After completion of the culture, XMP concentrations were measured using HPLC.

In order to convert the produced XMP into GMP, the conversion reaction was performed at 40°C for 2.5 hours by adding the following conversion reaction additives and *E*. *coli* XMP aminoase to the flask fermentation solution (Korean Patent No. 10-1210704). After completion of the reaction, the conversion rate, indicating the amount of GMP produced relative to the amount of XMP consumed, is shown in Table 7 below.

**[Table 7]**

| Name of strain | Concentration (g/L) | | Conversion rate (%) (amount of GMP produce/amount of XMP consumed) |
|---|---|---|---|
| | XMP | GMP | |
| CJX1664 | 4.60 | 3.33 | 72.3 |
| CJX2591 | 5.21 | 3.81 | 73.1 |
| CJX1682 | 12.49 | 8.93 | 71.5 |
| CJX2592 | 13.18 | 9.53 | 72.3 |

As shown in Table 7, it was confirmed that CJX2591 strain and CJX2592 strain showed 14.4% and 6.7% increase in the XMP-producing ability, respectively, as compared to the control CJX1664 and CJX1682 strains, and the GMP conversion rates of CJX2591 strain and CJX2592 strain were increased, as compared to those of the control CJX1664 and CJX1682 strains.

The CJX2591 and CJX2592 strains were deposited at the Korea Microorganism Conservation Center, a depository institution under the Budapest Treaty, on September 8, 2022, and given accession number KCCM13236P and KCCM13237P, respectively.

Based on the above description, it will be understood by those skilled in the art to which the present disclosure pertains that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

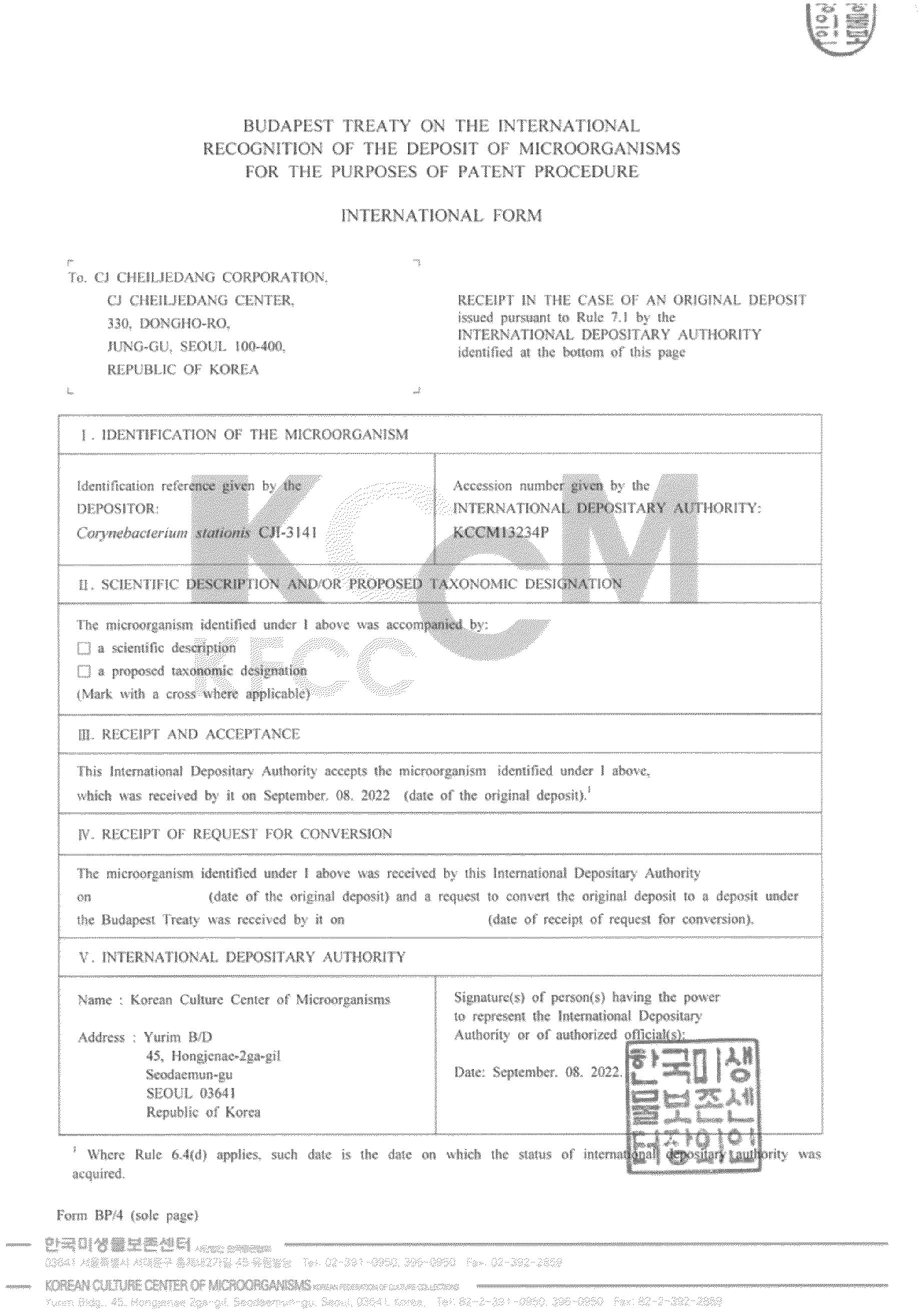

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium stationis CJI-3141 | Accession number given by the
INTERNATIONAL DEPOSITARY AUTHORITY:
KCCM13234P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 08. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 08. 2022.

¹ Where Rule 6.4(d) applies, such date is the date on which the status of international depositary authority was
acquired.

Form BP/4 (sole page)

한국미생물보존센터
03641 서울특별시 서대문구 홍제내2가길 45 유림빌딩 Tel: 02-391-0950, 396-0950 Fax: 02-392-2859
KOREAN CULTURE CENTER OF MICROORGANISMS
Yurim Bldg., 45, Hongjenae 2ga-gil, Seodaemun-gu, Seoul, 03641, Korea. Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium stationis CJI-3142 | Accession number given by the
INTERNATIONAL DEPOSITARY AUTHORITY:
KCCM13235P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 08. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 08. 2022.

¹ Where Rule 6.4(d) applies, such date is the date on which the status of international depositary authority was
acquired.

Form BP/4 (sole page)

한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS [?]
Yurim Bldg., 45, Hongjenae 2ga-gil, Seodaemun-gu, Seoul, 03641, Korea. Tel: 82-2-391-0950, 396-0950 Fax: 82-2-392-2859

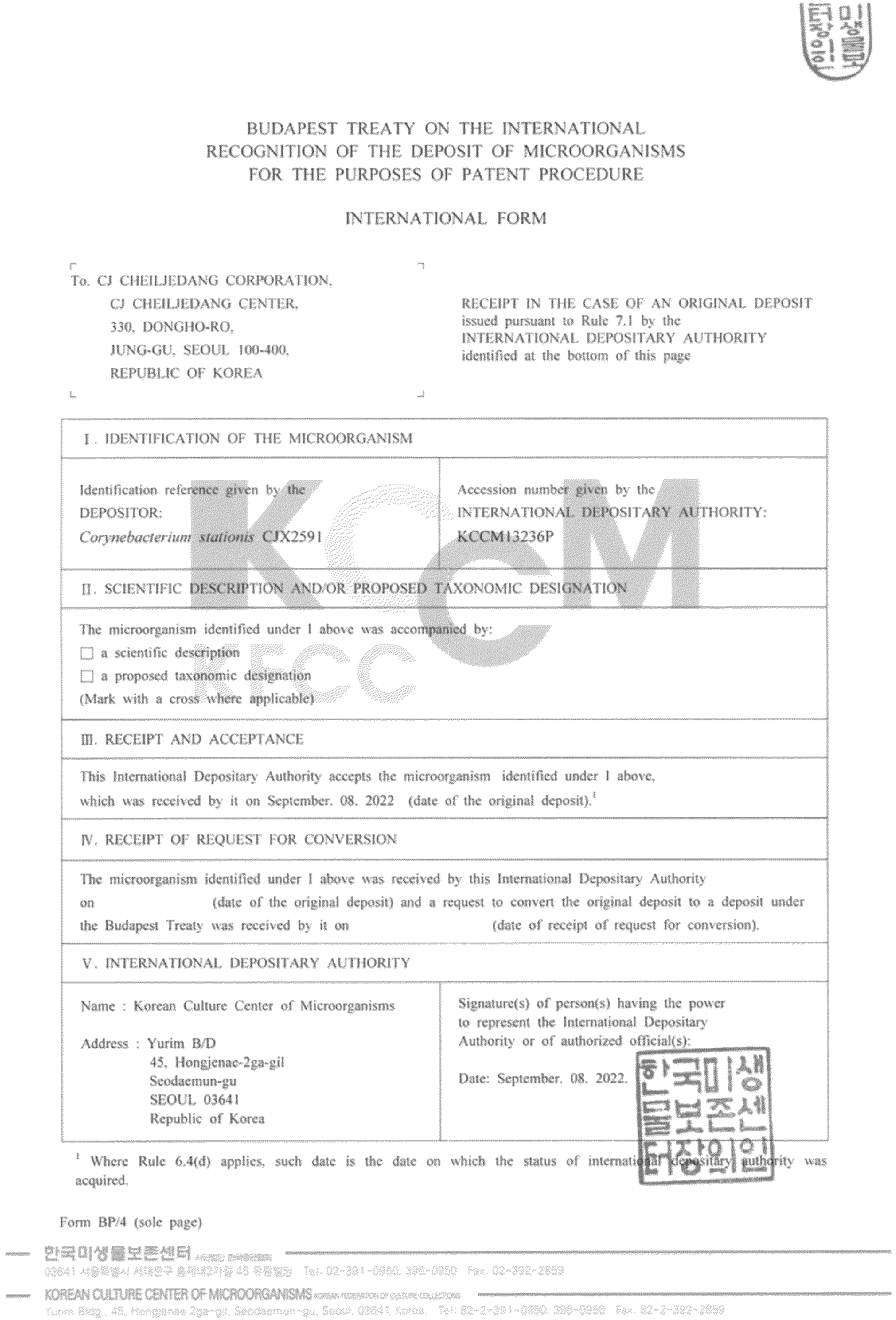

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium stationis CJX2591 | Accession number given by the
INTERNATIONAL DEPOSITARY AUTHORITY:
KCCM13236P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 08. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 08. 2022.

¹ Where Rule 6.4(d) applies, such date is the date on which the status of international depositary authority was
acquired.

Form BP/4 (sole page)

한국미생물보존센터
03641 서울특별시 서대문구 홍제내2가길 45 유림빌딩 Tel. 02-391-0950, 396-0950 Fax. 02-392-2859
KOREAN CULTURE CENTER OF MICROORGANISMS
Yurim Bldg. 45, Hongjenae 2ga-gil, Seodaemun-gu, Seoul, 03641, Korea. Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium stationis CJX2592 | Accession number given by the
INTERNATIONAL DEPOSITARY AUTHORITY:
KCCM13237P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 08. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 08. 2022.

¹ Where Rule 6.4(d) applies, such date is the date on which the status of international depositary authority was
acquired.

Form BP/4 (sole page)

한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS [?]
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. A purine nucleotide-producing microorganism of the genus *Corynebacterium,* wherein activity of an Mn transporter is enhanced.

2. The microorganism of claim 1, wherein the purine nucleotide is any one or more selected from the group consisting of IMP, XMP, and GMP.

3. The microorganism of claim 1, wherein the Mn transporter has an amino acid sequence of SEQ ID NO: 1 or an amino acid sequence having 70% or more homology thereto.

4. The microorganism of claim 1, wherein the microorganism of the genus *Corynebacterium* has enhanced activity of the Mn transporter by increasing expression of the Mn transporter, as compared to a wild-type microorganism of the genus *Corynebacterium.*

5. The microorganism of claim 1, wherein the microorganism of the genus *Corynebacterium* is *Corynebacterium stationis.*

6. A method of producing purine nucleotides, the method comprising the step of culturing, in a medium, a purine nucleotide-producing microorganism of the genus *Corynebacterium,* wherein activity of an Mn transporter is enhanced.

7. The method of claim 6, wherein the purine nucleotide is any one or more selected from the group consisting of IMP, XMP, and GMP.

8. A composition for producing purine nucleotides, the composition comprising a microorganism of the genus *Corynebacterium,* wherein activity of an Mn transporter is enhanced; a medium in which the microorganism is cultured; or a combination thereof.
